Europäisches Patentamt

European Patent Office

Office européen des brevets

(11) Publication number: **0 362 274 B1**

# EUROPEAN PATENT SPECIFICATION

(45) Date of publication of patent specification: **23.03.94**

(21) Application number: **88905161.1**

(22) Date of filing: **03.06.88**

(86) International application number:
**PCT/AU88/00176**

(87) International publication number:
**WO 88/09668 (15.12.88 88/27)**

(51) Int. Cl.5: **A61K 37/547**, A61K 39/02,
C12N 1/20, C12N 9/52,
C12N 15/00, C07K 15/12,
C12P 21/02, G01N 33/569,
G01N 33/573, G01N 33/566,
C07H 21/04

(54) **TREATMENT AND DIAGNOSIS OF FOOTROT USING THE BASIC PROTEASE OF BACTEROIDES NODOSUS.**

(30) Priority: **05.06.87 AU 2316/87**

(43) Date of publication of application:
**11.04.90 Bulletin 90/15**

(45) Publication of the grant of the patent:
**23.03.94 Bulletin 94/12**

(84) Designated Contracting States:
**AT BE CH DE FR GB IT LI LU NL SE**

(56) References cited:
**AU-A- 8 551 387**
**AU-B- 7 022 162**
**AU-B- 8 060 749**
**AU-B- 8 434 979**

**AUSTRALIAN JOURNAL OF BIOLOGICAL SCIENCES, vol. 35, 1982; A.A. KORTT et al., pp. 481-489&NUM;**

(73) Proprietor: **THE COMMONWEALTH SCIENTIFIC AND INDUSTRIAL RESEARCH ORGANIZATION**
**Limestone Avenue,**
**P.O. Box 1600**
**Canberra, Australian Capital Territory 2601(AU)**

(72) Inventor: **STEWART, David, James**
**11 Hastings Road**
**Hawthorn East, VIC 3123(AU)**
Inventor: **KORTT, Alexander, Andrew**
**23 Upland Road**
**Strathmore, VIC 3041(AU)**
Inventor: **LILLEY, Glenn, George**
**11 Arthur Street**
**Doncaster, VIC 3108(AU)**

JOURNAL OF GENERAL MICROBIOLOGY, vol. 131, 1985, GB; R.S. GREEN, pp. 2871-2876&NUM;

VETERINARY MICROBIOLOGY, vol. 12, 1986, Amsterdam (NL); W.K. YONG et al., pp. 135-145&NUM;

VETERINARY MICROBIOLOGY, vol. 9, 1984, Amsterdam (NL); L.J. DEPIAZZI et al., pp. 227-236&NUM;

RESEARCH IN VETERINARY SCIENCE, vol. 35, 1983; A.A. KORTT et al., pp. 171-174&NUM;

JOURNAL OF GENERAL MICROBIOLOGY, vol. 128, 1982, GB; D. EVERY, pp. 809-812&NUM;

RESEARCH IN VETERINARY SCIENCE, vol. 39, 1985; pp. 165-172&NUM;

(74) Representative: **Geering, Keith Edwin et al**
**REDDIE & GROSE**
**16 Theobalds Road**
**London WC1X 8PL (GB)**

**Description**

This invention relates to a prophylactic and therapeutic treatment of ovine footrot and in particular relates to vaccine effective in such treatment of ovine footrot. The invention also relates to the diagnosis of ovine footrot.

Ovine footrot is a contagious disease of the sheep's foot which is characterised by separation of the hoof from the living epidermal tissues due to a spreading infection in the interdigital skin and beneath the horn. The essential causative agent of ovine footrot infection is the anaerobic Gram-negative bacterium Bacteroides nodosus. The disease is of considerable economic significance throughout the temperature areas of the world where sheep are raised. In Australia alone the estimated losses due to footrot amount to millions of dollars annually.

Classification of strains of B.nodosus in serogroups and subgroups is based on agglutinogens which are present on surface filaments termed pili or fimbriae. The pili induce a high level of protection in vaccinated sheep against homologous serogroups of B.nodosus (Stewart 1978, Every and Skerman 1982; Stewart et.al. 1982, 1983a, 1983b, 1986a). Since there are at least 8 serogroups, the current commercial vaccines contain killed whole cells of 8-10 well piliated strains representative of all the known serogroups (Claxton et.al. 1983). These vaccines are more expensive than other vaccines used in sheep partly due to the need for the incorporation of a multiple number of strains in the vaccine and partly due to the fastidious growth requirements and relatively sparse growth of B.nodosus in liquid media. Pilus expression tends to be variable in B. nodosus and, especially with some strains, the irreversible loss of pili from cells following subculture in liquid medium is a major problem to manufacturers.

Although homologous protection is superior to that obtained against heterologous strains, nevertheless some cross-protection against heterologous serogroups has been obtained with whole cell vaccines (Thorley and Egerton 1981; Stewart et.al. 1983a; Stewart et.al. 1985; Stewart et.al. 1986a). The identification of the nature and location of the cross-protective antigens may facilitate the rational selection of a reduced number of vaccine serotypes based on the presence of serogroup-specific pilus antigens and cross-protective non-pilus antigens, the latter being common to (shared by) at least some serogroups. Alternatively, vaccination of sheep with large amounts of cross-protective antigens may enable a high level of protection to be induced against a wide range of serogroups.

One group of cross-protective antigens is described in prior Australian Patent Application No.51387/85. This application describes a group of extracellular serine protease isoenzymes elaborated in B.nodosus culture supernatants which induce protection against the homologous strain of B.nodosus from which the isoenzymes were derived as well as heterologous strains in different serogroups. The purification and properties of these protease isoenzymes have also been described by Kortt et.al. (1986), and their effectiveness as vaccines for preventing footrot by Stewart et.al. (1986b). These isoenzymes thus form the basis for effective prophylactic and therapeutic treatment against ovine footrot.

Polyacrylamide gel electrophoresis (PAGE) of B.nodosus culture supernatants has shown the presence of a mixture of isoenzymes which can be separated into 4 to 5 unique bands (Every 1982; Kortt et.al. 1982), on the basis of differences in ionic charges present, on 5 to 7% polyacrylamide gels using the non-dissociating buffer system of Davis (1964). The protease isoenzymes are detected by their activity using the zymogram technique as described (Kortt et.al. 1983). The protease isoenzymes detected by the zymogram technique account for the bulk of the proteolytic activity present in the cultures and this activity elutes as a major broad peak, at a Ve/Vc (Volume of eluant/Volume of column) ratio of approximately 0.6, on Sephadex G-100 The purification and properties of these protease isoenzymes are described in Australian Patent Application No. 51387/85.

It has now been discovered that a previously unknown basic (pI about 9) serine protease, differing markedly from any proteases disclosed previously, can be isolated from either virulent or intermediate strains of B.nodosus, and that this basic serine protease can also be used to induce protection and a curative response against footrot induced by the homologous strain from which the basic serine protease is derived, as well as strains in different heterologous serogroups. Hereinafter "the basic serine protease" of B.nodosus is to be understood to be of pI about 9.

Thus according to one aspect of the present invention there is provided a vaccine for use in protection against or treatment of footrot, the vaccine containing an immunologically effective amount of the basic serine protease of Bacteroides nodosus as the active component thereof, together with an acceptable pharmaceutical or veterinary diluent or carrier therefor. If desired, an adjuvant may be included in the vaccine.

In yet another aspect of the present invention, there is provided a method for the detection of footrot, and in particular for detecting the presence of virulent or intermediate strains of B.nodosus, which uses the

3

basic serine protease of B.nodosus as an antigen in an immunoassay. Such an immunoassay may, for example, be directed to the detection of antibodies to the basic protease in sheep serum. Full details of the methods by which such immunoassays can be carried out are well known in the art, and are accordingly not described in detail here. These methods include, for example, the well-known ELISA and radioimmunoassays.

Since the basic protease described herein appears to be produced by virulent and intermediate strains of B.nodosus, but not by benign strains, the invention also extends to diagnostic tests to detect the presence of such virulent or intermediate strains both in vivo, for example in lesion material, and in vitro, for example in broth cultures of lesion material. Such diagnostic tests may be based on the use of monoclonal antibodies produced to the basic protease, or on the use of oligonucleotide or DNA or RNA probes corresponding to basic protease gene sequences which appear to be unique to these virulent and intermediate strains.

The present invention further provides antibodies, particularly monoclonal antibodies, (including both mouse and sheep monoclonal antibodies) to the basic serine protease of B.nodosus, These antibodies have particular utility in diagnostic tests as described above. The production of such antibodies, both polyclonal and monoclonal, is well known in the art.

The basic serine protease of B.nodosus used as the active component of a vaccine in accordance with a present invention may be used as the purified protease, as a partially purified culture supernatant, or as a concentrated crude culture supernatant of B.nodosus. In yet another aspect, the basic serine protease may be produced through recombinant DNA technology which allows the transfer of the genes encoding the protease of B.nodosus into a new host in which the genes are capable of being expressed to produce large amounts of the desired protease.

In this aspect of the invention there is provided a recombinant DNA molecule comprising a nucleotide sequence capable of being expressed as all or at least a substantial part of the basic serine protease of B.nodosus, or a peptide comprising at least one epitope thereof. The nucleotide sequence may have expression control sequences positioned adjacent to it, such control sequences being derived from a homologous or heterologous source. In this aspect, the invention also provides a recombinant DHA cloning vehicle comprising an expression control sequence and a nucleotide sequence capable of being expressed as a polypeptide having the antigenicity of the basic serine protease, and a host cell containing such a cloning vehicle.

As described above, the present invention also extends to synthetic polypeptides displaying the antigenicity of the basic serine protease. Such synthetic polypeptides may comprise fusion polypeptides wherein the sequence displaying the desired antigenicity is fused to an additional polypeptide. As used herein, the term "synthetic" means that the polypeptides have been produced by chemical or biological means, for example by chemical peptide synthesis or by recombinant DNA techniques leading to biological synthesis.

The diluent or carrier may be any suitable pharmaceutical or veterinary diluent or carrier, the exact form of which will depend on the administration technique to be used to obtain effective vaccination. The means of administration may be oral, subcutaneous, intravenous, intramuscular, intraperitoneal, by aerosol, suppository, bolus or by any other suitable procedure. The vaccine may include a known adjuvant, such as an oil, aluminium hydroxide, saponin-aluminium hydroxide, or aluminium hydroxide-oil adjuvant, to improve its efficacy. The adjuvant may itself be the carrier, and the adjuvant/carrier/means of administration will be chosen to enhance the antibody response. One advantage of the use of the present vaccine is that the size of the granulomatous reaction at the site of a subcutaneous inoculation may be substantially reduced when an oil-adjuvant vaccine contains purified antigens rather than whole organisms.

Purification and Characterisation of the Basic Protease.

In the work leading to the present invention, a second minor peak of proteolytic activity, eluting near the void volume of the Sephadex G-100 column (150x4cm) was found on gel filtration of concentrates of bulk cultures of virulent and intermediate strains of B.nodosus. This minor peak of proteolytic activity eluted well before the bulk of the proteolytic activity eluting at Ve/Vo of 0.6. The active fractions of this (high molecular weight) peak were pooled, concentrated and dialysed against 0.01M Tris HCl-5 mM $CaCl_2$ pH 8.5 at 4°C. This material was then chromatographed on a sulfo-propyl (SP)-Sephadex C-25 column (20x2cm) equilibrated with 0.01M Tris HCl-5mM $CaCl_2$ pH 8.5. The proteolytic activity which bound to the SP-Sephadex C-25 was eluted with a 0-0.3M NaCl gradient at 4°C. A single protein peak with proteolytic activity (as assessed with hide powder azure substrate) was eluted at approx. 0.2M NaCl. This protease peak, referred to herein as "basic protease" was concentrated and its purity assessed by non-dissociating polyacrylamide gel electrophoresis at pH 4.3 (Reisfeld et.al. 1962) and pH 3.1 (du Cros and Wrigley 1974). The basic protease migrated as a single component under these conditions indicating that it is homogeneous.

The high isoelectric point of this basic protease (pI ~ 9.0) precludes its electrophoresis at pH 8.3 and hence its observation on zymograms as used to visualize the isoenzymes which have isoelectric points in the range 4.9 to 6.0. Therefore this basic protease is not observed on zymograms of crude bulk cultures of B.nodosus and the occurrence of this enzyme in these cultures has not been previously recognized.

On SDS-PAGE gels the basic protease migrated as a single protein band with an apparent molecular weight of approximately 37,000. Gel filtration studies using a calibrated Sephadex G-150 column (150x1cm) yielded a molecular weight of approx. 150,000 for the native protease indicating that it is a tetramer composed of four subunits of MW 37,000.

The basic protease is completely inhibited by phenyl methylsulfonyl fluoride (PMSF) and diisopropyl fluorophosphate (DFP) indicating that it is a serine protease. The basic protease is also inhibited by the metal ion chelator, EDTA; on adding EDTA the protease loses activity by undergoing rapid autolysis. Furthermore, the basic protease, inactivated with PMSF, in the presence of calcium resists degradation by other proteases such as α-chymotrypsin; however, chelation of the calcium with EDTA renders the PMSF inactivated protease susceptible to proteolytic degradation. Thus the divalent metal ion, calcium, stabilizes the protease structure and protects it from autolysis and/or degradation by proteolysis. The incorporation of calcium is therefore essential to stabilize the protease antigen in vaccine formulations.

The basic protease digests hide powder azure, gelatin, casein and elastin. It also digests extensively peptide substrates such as the A- and B-chains of insulin and glucagon. Basic protease cleaved the S-carboxymethyl B-chain of insulin at the following peptide bonds; Asn-Gln, His-Leu, Leu-CMCys, Ser-His, Leu-Tyr, CMCys-Gly, Arg-Gly, Phe-Phe, and Phe-Tyr, while the CMCys-Ser and Leu-Glu bonds were cleaved in S-carboxymethyl A-chain of insulin. The bonds cleaved in glucagon were; Gln-Gly, Thr-Phe, Phe-Thr, Ser-Asp, Tyr-Ser, and Gln-Asp. These results demonstrate that the basic protease does not show any preference for cleavage at specific residues in these peptides and may therefore be described as a non-specific protease.

The basic protease also hydrolyses α-N-benzoyl-L-tyrosine ethyl ester, a substrate used to assay α-chymotrypsin and subtilisin, and N-succinyl-L-Ala-Ala-Ala-p-nitroanilide a substrate used to assay elastase.

Basic protease has been isolated from B.nodosus cultures of virulent strains 198, 234,265, and the intermediate strain 332. Benign strain 305, produces a small amount of a protease which elutes at 0.1M NaCl from SP-Sephadex C-25 as described above with similar antigenic properties, however it did not contain a protease with the same isolelectric point as that for virulent strains. The amino acid compositions of the basic protease from strain 198, 234, 265 and 332 are very similar and the compositions of the basic protease from virulent strain 198 and intermediate strain 332 are presented in Table 1.

TABLE 1

| "Amino acid compositions of basic proteases isolated from 2 strains of B.nodosus[1]. | | | | | |
|---|---|---|---|---|---|
| | Residues % | | Residues/ 350 residues | | Sequence composition (198)[2] |
| | 198 | 332 | 198 | 332 | |
| LYS | 3.8 | 3.9 | 13.3 | 13.7 | 12 |
| HIS | 2.1 | 2.2 | 7.3 | 7.7 | 7 |
| ARG | 5.3 | 5.3 | 18.6 | 18.6 | 19 |
| ASP | 14.3 | 15.2 | 50.1 | 53.2 | 52 |
| THR | 6.3 | 5.6 | 22.1 | 19.6 | 20 |
| SER | 9.2 | 7.6 | 32.2 | 26.6 | 27 |
| GLU | 6.4 | 5.2 | 22.4 | 18.2 | 12 |
| PRO | 6.3 | 5.9 | 22.1 | 20.7 | 17 |
| GLY | 11.9 | 12.4 | 41.7 | 43.4 | 47 |
| ALA | 9.3 | 10.3 | 32.6 | 36.1 | 36 |
| 1/2CYS | 1.1 | 0.6 | 3.9 | 2.1 | 4 |
| VAL | 8.5 | 8.9 | 30.0 | 31.2 | 33 |
| MET | ND | ND | - | - | 6 |
| ILE | 4.3 | 4.1 | 15.1 | 14.4 | 15 |
| LEU | 5.5 | 5.4 | 19.3 | 18.9 | 19 |
| TYR | 2.5 | 3.1 | 8.8 | 10.9 | 10 |
| PHE | 2.2 | 2.4 | 7.7 | 8.4 | 7 |
| TRP | ND | ND | - | - | 7 |
| Total | | | | | 350 |

1. Samples of basic protease (100μg) were hydrolysed in 6M HCl, 110°C, 24h. The values are uncorrected. ND = not determined.

2. Composition from the sequence of the basic protease (Table 2).

Cloning and Characterisation of the Basic Protease Gene.

The clones containing the basic protease gene were obtained from the genomic library of B.nodosus strain 198 prepared by Elleman et.al. (1984). The genomic library was constructed by Sau3AI digestion of extracted and purified chromosomal DNA, and the DNA fragments were then ligated to BamHI-cleaved, dephosphorylated pBR322 and the mixture used to transform E.coli strain RRI. Recombinant clones sensitive to tetracycline were grown to saturation in the presence of 50μg/ml ampicillin in individual wells of microtitre plates. The cultures were stored frozen at -70°C until used. Recombinant clones were screened on YT agar plates [0.5% yeast extract (Difco), 0.8% Bacto Tryptone (Difco), 0.5% NaCl and 1.6% Bacto agar (Difco)], containing 50μg/ml ampicillin and a proteolytic substrate, 10% skim milk powder. The plates were incubated for a minimum of 72 hours. Two protease-producing clones were obtained following screening of about 1000 colonies, and these were detected, after about 30 hours of incubation, by zones of clearing underneath and around the 2 positive colonies, (clones 21G5 and 19G2). Both positive colonies were used for preparing protease supernatants and in each case 100 YT plates containing ampicillin and skim milk powder (YTASM agar) were separately seeded to give confluent growth on the plates. The seed culture was grown overnight in 5ml YT broth containing 50μg/ml ampicillin and 10% skim milk powder, and stored at -20°C in the presence of 50% sterile glycerol until required. Far 21G5 the seed culture was grown overnight in YT broth containing ampicillin, and stored at -7°C until used. The plates were harvested after 63 to 65 hours (19G2) or 21 hours (21G5) of incubation when clearing of the skim milk by protease was complete. The 19G2 and 21G5 protease supernatants were extracted from YTASM agar by high speed centrifugation at 23,000xg for 60 minutes and the supernatant were clarified by additional centrifugation at 23,000xg for 60 minutes. The supernatants were then concentrated 10 fold in an Amicon ultrafiltration cell using a Diaflo YM10 membrane.

The concentrated supernatants prepared from E.coli clones 19G2 and 21G5 showed complete identity with the native basic protease in agar gel diffusion using an antiserum raised in sheep to the native basic protease. Immunoblotting from SDS-PAGE gels confirmed that the cloned gene produced a protein that was similar to the native basic protease in both molecular weight and antigenicity.

Additional evidence that clone 19G2 contained the basic protease gene was obtained, firstly by subcloning into a bacterial expression vector. The pBR322 plasmid containing a 11.5kb insert was purified from clone 19G2 on a cesium chloride gradient, digested with Sau3AI and the fragments were ligated into the BamHI site of a pEX 1/2/3 plasmid vector. This expression vector was used to transform E.coli POP2136. Subclones were screened with a colony immunoassay (Elleman et.al 1984), to detect expression of ß-galactosidase fusion proteins containing epitopes of the basic protease, using polyclonal antiserum prepared in sheep against the native basic protease, horse radish peroxidase labelled donkey antisheep IgG (Silenus Laboratories Pty.Ltd.) and 4-chloro-1-naphthol as substrate. Immunoblotting using the same enzyme catalysed colour reaction detection system confirmed that 6 subclones out of 100 expressed a fusion protein containing epitopes recognised by the basic protease antiserum.

Secondly, direct evidence that the protease expressed by E.coli clones 19G2 and 21G5 is identical to the native basic protease isolated from B.nodosus was established by direct amino acid sequencing of the basic protease and the sequencing of protease gene isolated from clones 19G2 and 21G5.

An oligonucleotide probe was constructed based on the sequence Asp-Glu-Gly-Asp-Trp-Phe-Asn determined by direct sequencing of a tryptic peptide. Using the preferred codon usage for B.nodosus - (Elleman and Hoyne, 1984) a 23 mer oligonucleotide probe was synthesized with a minimum of redundancy. The probe consisted of a mixture of four oligonucleotide sequences with 2 substitutions at two positions (9 and 18) containing alternate nucleotides. The nucleotide sequences in the probe were:

$$
\begin{array}{ccc}
1 & 9 & 18 \\
& T & C \\
\text{GATGAAGG} & \text{GATTGGTT} & \text{GATAA} \\
& A & T
\end{array}
$$

The pBR322 plasmids from E.coli clones 19G2 and 21G5, containing the 11.5 kb and 11 kb insert respectively from B.nodosus strain 198, which expressed the basic protease activity, were digested with several restriction enzymes (HindIII, AccI, ClaI, EcoRV and EcoRI) and probed with the synthetic oligonucleotide described above. A single fragment in each restriction enzyme digest was shown to hybridize strongly with the probe showing a high degree of specificity of the probe. Preliminary sequencing of the respective DNA fragments from both clones revealed complete identity. Clone 21G5 was subsequently used for determining the complete nucleotide sequence of the basic protease gene.

A HindIII/EcoRI fragment, identified from clone 21G5 by hybridization to the oligonucleotide probe described above was force cloned into the sequencing vectors M13-Mp18 and M13-Mp19 for sequencing by the dideoxy chain termination method. Synthetic primers were produced which enabled the entire sequence to be determined in both insert orientations. This fragment was found to contain an open reading frame of approximately 1960 bases which coded for the complete sequence of the basic protease including a hydrophobic signal and a pro-sequence.

To obtain direct amino acid sequence information, the basic protease from B.nodosus strain 198 was reduced and alkylated with iodoacetic acid and digested with trypsin. The tryptic peptides were separated and purified by RP-HPLC and the peptides were sequenced either with a gas-phase sequencer or manually as described by Kortt et.al. (1987). The amino-terminal sequence of intact basic protease was determined using the gas-phase sequencer.

The complete nucleotide sequence of the B.nodosus basic protease gene is presented in Table 2. The deduced amino acid sequence is shown in the single letter code notation. The sequence of the amino acid residues determined by direct peptide sequencing of peptides derived from the tryptic digest of the basic protease is underlined in Table 2. The junction between the pro-peptide and the mature amino-terminus is shown by the arrow. The carboxy-terminal residue of the mature protein remains to be characterised to determine whether carboxy-terminal processing occurs.

The amino acid composition calculated from the sequence (Table 2) is in close agreement with the amino acid composition determined for the protease (Table 1). These results demonstrate unequivocally that the protease expressed by E.coli clone 21G5 is identical to the native basic protease isolated from B.nodosus.

The protein and gene sequencing data show that the basic protease of B.nodosus is composed of about 350 amino acid residues and has a Mr ~38,500 in agreement with the value found on SDS-PAGE. Analysis of the nucleotide sequence of the gene shows that the protease is synthesized as a preproprotein (Mr ~ 50,000) which is processed after synthesis to produce the mature protease isolated from the

7

extracellular medium of B.nodosus cultures. Other bacterial extracellular proteases (e.g. subtilisn from Bacillus subtilis) are synthesized and processed in a similar manner.

TABLE 2: Nucleotide (gene) and deduced amino acid sequence
of the basic protease (including pre-pro sequence)
from Bacterioides nodosus.

```
  1 agc gca ttg gag cgc ggt ttg ccc gat tgc gca ggc gtt gcg ctc ggc

 49 gtg gat cgg tta ttc atg tta gcg caa gga aaa aac cat att gcg gaa

 97 gtg att tta taa acg aat aaa tta ttt tat cga taa tat ttt tga taa

145 ttt ttt gaa aaa taa caa ata ttt ttt att aac gca gtg att ttt gat

193 taa tta ata taa tta ata taa cca ttt ttt aaa aaa ttt ttt tgc tat

241 tta tct ttc att tac gta ata tac tca gtg ctg aat aaa taa aaa tta

                                  M   K   S   I   T   G   E   S   M   N
289 att att tag caa tta tta ATG AAA TCA ATA ACA GGT GAA AGC ATG AAT

        L   S   N   I   S   A   V   K   V   L   T   L   V   V   S   A
337 CTA TCG AAC ATT TCT GCG GTA AAA GTA TTA ACA CTG GTG GTT AGC GCT

        A   I   A   G   Q   V   C   A   A   E   S   I   V   N   Y   E
385 GCC ATC GCT GGT CAA GTT TGC GCG GCT GAA AGT ATC GTC AAC TAT GAA

        S   A   N   A   I   S   K   Q   P   E   G   S   V   R   F   I
433 TCA GCA AAT GCC ATT AGC AAA CAG CCA GAA GGC AGT GTG CGC TTT ATC

        V   K   Y   K   D   G   T   P   S   S   Q   G   L   K   T   R
481 GTC AAA TAT AAA GAT GGC ACG CCA TCA AGC CAA GGT TTG AAA ACA CGC

        S   T   T   K   V   M   A   S   G   M   Q   V   V   G   F   E
529 AGC ACA ACT AAA GTA ATG GCA AGC GGT ATG CAA GTT GTT GGT TTT GAA

        A   Q   F   V   R   T   T   G   L   G   A   G   I   F   A   V
577 GCA CAA TTT GTG CGC ACA ACC GGT TTA GGC GCG GGC ATT TTT GCT GTA

        P   E   L   K   T   T   K   E   A   H   L   V   M   D   T   I
625 CCA GAA TTA AAA ACA ACC AAA GAA GCG CAT TTG GTG ATG GAT ACC ATT

        A   S   N   P   D   V   E   F   V   E   V   D   R   L   A   Y
673 GCT TCT AAT CCT GAT GTT GAA TTT GTG GAA GTT GAC CGT TTA GCG TAT

        P   K   A   A   P   N   D   P   S   Y   R   Q   Q   W   H   Y
721 CCA AAA GCT GCG CCT AAT GAT CCG AGC TAT CGA CAA CAA TGG CAT TAT

        F   G   N   Y   G   V   K   A   N   K   V   W   D   R   G   F
769 TTC GGC AAT TAC GGC GTT AAA GCT AAT AAA GTT TGG GAT CGC GGA TTT

        T   G   Q   G   V   V   V   S   V   V   D   T   G   I   L   D
817 ACC GGT CAA GGC GTA GTT GTG AGC GTC GTG GAT ACT GGA ATT TTG GAT

        H   V   D   L   N   G   N   M   L   P   G   Y   D   F   I   S
865 CAC GTT GAT TTA AAT GGA AAT ATG TTG CCA GGT TAT GAC TTT ATC TCT

        S   A   P   N   A   R   D   G   D   Q   R   D   N   N   P   A
913 AGT GCG CCC AAT GCC CGA GAT GGA GAT CAA CGC GAT AAT AAT CCT GCC
```

8

Table 2 (cont'd)

```
      D   E   G   D   W   F   D   N   W   D   C   G   G   Y   P   D
961   GAC GAA GGC GAT TGG TTT GAT AAT TGG GAT TGC GGT GGT TAC CCT GAT

      P   R   R   E   K   K   F   S   T   W   H   G   S   H   V   A
1009  CCG CGC AGA GAA AAA AAA TTC AGC ACT TGG CAC GGC TCT CAC GTT GCT

      G   T   I   A   A   V   T   N   N   G   V   G   V   A   G   V
1057  GGA ACC ATT GCC GCG GTA ACC AAT AAC GGC GTT GGC GTT GCA GGC GTT

      A   Y   G   A   K   V   I   P   V   R   V   L   G   K   C   G
1105  GCT TAT GGC GCG AAA GTA ATT CCG GTA CGC GTA TTA GGA AAA TGC GGC

      G   Y   D   S   D   I   T   D   G   M   Y   W   S   A   G   G
1153  GGT TAT GAT TCT GAT ATT ACA GAC GGA ATG TAT TGG TCA GCA GGC GGT

      H   I   D   G   V   P   D   N   Q   N   P   A   Q   V   V   N
1201  CAT ATT GAC GGC GTT CCT GAT AAC CAA AAT CCT GCT CAA GTA GTT AAT

      M   S   L   G   G   G   G   C   S   T   K   L   T   R   M
1249  ATG AGT TTA GGC GGC GGT GGC GGT TGT TCA ACA AAA CTC ACA CGT ATG

      I   D   K   T   T   N   L   G   A   L   I   V   I   A   A   G
1297  ATT GAC AAA ACC ACT AAT TTA GGT GCA TTA ATC GTT ATC GCT GCC GGT

      N   E   N   Q   D   A   S   R   T   W   P   S   S   C   N   N
1345  AAT GAA AAT CAA GAT GCC AGC AGA ACA TGG CCG AGC AGT TGT AAT AAC

      V   L   S   V   G   A   T   T   P   K   G   K   R   A   P   F
1393  GTA TTA AGC GTT GGC GCA ACC ACA CCA AAA GGA AAA CGC GCT CCA TTT

      S   N   Y   G   A   R   V   H   L   A   A   P   G   T   N   I
1441  TCA AAC TAC GGC GCC CGA GTT CAT TTA GCC GCA CCG GGT ACA AAT ATT

      L   S   T   I   D   V   G   Q   A   G   P   V   R   S   S   Y
1489  TTA TCA ACT ATT GAC GTT GGA CAA GCA GGA CCT GTT AGA AGC AGT TAC

      G   M   K   A   G   T   S   M   A   A   P   H   V   S   G   V
1537  GGC ATG AAA GCC GGT ACA TCA ATG GCG GCT CCG CAT GTT TCT GGC GTT

      A   A   L   V   I   S   A   A   N   S   I   G   K   T   L   T
1585  GCA GCG TTA GTG ATT TCT GCG GCA AAT AGT ATC GGT AAA ACT TTA ACA

      P   S   E   L   S   D   I   L   V   R   T   T   S   R   F   N
1633  CCG TCA GAA TTA AGC GAT ATT TTA GTA CGC ACT ACC AGC CGT TTT AAT

      G   R   L   D   R   G   L   G   S   G   I   V   D   A   N   A
1681  GGT CGT TTA GAT CGG GGA TTG GGA TCA GGT ATT GTT GAT GCC AAT GCT

      A   V   N   A   V   L   G   D   Q   N   R   A   Q   P   R   P
1729  GCG GTT AAT GCT GTT TTA GGC GAT CAA AAT CGC GCT CAA CCT CGA CCG

      P   V   N   Q   P   I   N   S   G   N   K   V   Y   R   S   D
1777  CCG GTT AAT CAA CCC ATT AAT TCT GGA AAT AAA GTT TAT AGA AGC GAC

      R   R   L   P   F   A   I   *
1825  CGC AGG TTG CCA TTC GCG ATT taa gat cat aac cag cgg tat tcg tgt

1873  taa cga tca gca cgc gtt gga tct gcc aat att acg ttg act tta gac

1921  att cgt cag gcg atc gtt cac aat aag caa gtg gaa ctg tta
```

The following Examples illustrate the use of the basic serine protease in the preparation of vaccines and in the use of such vaccines for inducing both protection and cure in sheep against footrot.

## EXAMPLE 1

The experiment was conducted with penned sheep at the Maribyrnong Field Station. The Merino sheep were vaccinated three times Subcutaneously in the neck region with an interval of 30 and 22 days between injections. They were challenged 4 days after the third vaccination by the application of pure cultures of the homologous strain (198).

### A. Vaccines

1. Strain 198 basic protease 50 $\mu$g per dose. This protease was produced from bulk cultures of virulent strain 198 grown anaerobically at 37°C for 2 to 4 days in Trypticase-arginine-serine (TAS) broth containing 0.15% $CaCl_2$. After removal of the cells by centrifugation, the culture supernatant was concentrated on a Diaflo spiral-wound ultrafiltration membrane cartridge Model SIYl0 (Amicon mol.wt. cut-off about 10,000) and in an Amicon ultrafiltration cell using a Diaflo YMI0 membrane (mol.wt. cut-off about 10,000). The concentrated supernatant was fractionated by gel filtration on Sephadex G-100, followed by ion-exchange chromatography of the (high molecular weight) proteolytic activity peak eluting near the column void volume on SP-Sephadex C-25 using the conditions described earlier. The proteolytic activity, eluted from the SP-Sephadex C-25 column with the salt gradient, contained the basic protease and was horogeneous as judged by PAGE. The basic protease vaccines in examples 1 and 2 were both produced from this product.

2. Strain 198 mixture of purified protease isoenzymes 50 $\mu$g per dose. The isoenzymes were produced as described previously in Australian Patent Application No. 51387/85.

3. Escherichia coli strain RRI cloned B.nodosus basic protease 29$\mu$g per dose. The preparation of the concentrated protease supernatant from clone 19G2 grown on 100 YT plates containing ampicillin and skim milk powder is described above.

4. Unvaccinated controls.

Vaccines 1 and 2 were diluted in either PBS pH7.2 (first vaccination) or 0.01 M Tris 5 mM $CaCl_2$ pH8.0 (second and third vaccination). The amount of protein in these two vaccines was estimated by absorbance at 280 nm assuming an extinction coefficient of 10. The extinction coefficient is defined as the absorbance of a 1% solution measured in a 1cm spectrophotometer cell. The results by this method are consistent with those obtained by the Lowry method as modified by Hartree (1972). Amount of basic protease in vaccine 3 was estimated by a sandwich ELISA as described in Example 4. Purified strain 198 basic potease was used as a standard. The aqueous phase of vaccines No.1 and 2, mixed with an equal volume of Alhydrogel, was emulsified with incomplete Freund's adjuvant (Difco) in the ratio 1:2. For the cloned protease vaccine (No.3) 0.5% formalin was added to the aqueous phase and Alhydrogel was not included in the oil adjuvanted vaccine.

### B. Experimental Design

Thirty six Merino sheep were ranked on initial body weight and randomly distributed in 4 equal groups each of 9 sheep. The sheep in each group were randomly allocated to 3 pens prior to challenge so that each pen contained 12 sheep (3 from each group). Each foot of each sheep was challenged by the predisposition and culture application method of Egerton and Roberts (1971) as modified by Stewart et.al. (1985).

Inspections of feet were carried out at 24, 45 and 60 days after challenge and the lesions evaluated by the scoring system of Stewart et.al. (1982, 1983b). Sheep were bled for serology prior to each vaccination and then at each inspection.

### C. Results

Both the basic protease and mixture of isoenzymes provided substantial protection against the homologous strain in comparison to the unvaccinated controls (Table 3). In the group vaccinated with cloned protease there was a reduction in the number of feet affected with severe footrot compared to the controls.

TABLE 3

| Percentage of feet affected with footrot in vaccinated groups of penned sheep artificially challenged with pure cultures of the homologous strain (198) 4 days after the third vaccination. | | | |
|---|---|---|---|
| Vaccine Group | % with footrot (days after challenge) | | |
| | 24 | 45 | 60 |
| 1. Basic protease | 5.6* (50)† | 19.4 (27.8) | 19.4 (19.4) |
| 2. Mixture of protease isoenzymes | 5.6 (50) | 8.3 (8.3) | 8.3 |
| 3. Cloned basic protease | 44.4 (100) | 62.5 (84.4) | 75 (87.5) |
| 4. Unvaccinated controls | 69.4 (97.2) | 86.1 (94.4) | 75 (80.6) |

\* scores ≧ 3c (severe footrot)
† scores 2-4 (all categories of footrot)

Pilus agglutinating antibody titres to strain 198 (Table 4) were similar in all groups at challenge but 45 days later mean titres were more elevated in the controls and the vaccine group receiving the cloned protease than in those receiving either native basic protease or the mixture of protease isoenzymes. This was due to the much higher level of infection in groups 3 and 4.

TABLE 4

| Mean homologous (strain 198) pilus agglutinating antibody titres of vaccinated groups of penned sheep | | | |
|---|---|---|---|
| Vaccine group | Agglutinin titres (days after challenge) | | |
| | 0 | 24 | 45 |
| 1. Basic protease | 22 | 86 | 148 |
| 2. Mixture of protease isoenzymes | 19 | 86 | 148 |
| 3. Cloned basic protease | 25 | 127 | 587 |
| 4. Unvaccinated controls | 30 | 173 | 549 |

At both the second and third vaccination, basic protease ELISA antibody titres were more elevated in the group receiving native basic protease than in those receiving either a mixture of protease isoenzymes or cloned protease (Table 5). This greater antibody response in the basic protease group was associated with specific basic protease precipitin antibodies as determined by agar gel diffusion and these precipitin antibodies were also present in the sera from the sheep in the cloned protease group. In the group vaccinated with the mixture of protease isoenzymes basic protease precipitin antibodies were only evident after the third vaccination. The mixture of protease isoenzymes induced specific precipitin antibodies distinct from those induced by the basic protease. The basic protease preparation contained a precipitating antigen distinct from the antigens (at least 2) present in the mixture of isoenzymes

TABLE 5

| Mean ELISA antibody titres to the strain 198 basic protease in vaccinated groups of penned sheep. | | |
|---|---|---|
| Vaccine group | ELISA antibody titres (days after challenge) | |
| | 0 | 24 |
| 1. Basic protease | 4,967 | 8,685 |
| 2. Mixture of protease isoenzymes | 64 | 1,531 |
| 3. Cloned basic protease | 120 | 686 |
| 4. Unvaccinated controls | <25 | <25 |

The basic protease from strains 198, 234, 265 and 332 in 4 separate serogroups (AI, BI, HI and I respectively) possessed common antigenic determinants as determined by agar gel diffusion and ELISA. The geometric mean ELISA antibody titres of 6 sheep vaccinated with 50μg of strain 198 (AI) basic protease in AI hydrogel-oil adjuvant were 10,888, 22,975, 13,791 and 17,926, respectively. Agar gel immunodiffusion also demonstrated that the strain 198 basic protease was antigenically distinct from the protease isoenzymes from the same strain.

EXAMPLE 2

The experiment was conducted on irrigated pasture at the CSIRO Werribee Field Station utilizing flood irrigation to provide sufficient moisture for pasture growth and transmission of footrot. One hundred and twenty mature Merino ewes were ranked on body weight and randomly distributed into 8 equal groups of 15. Four donors infected with pure cultures of B.nodosus strain 198 (serogroup AI) by the method of Egerton and Roberts (1971) as modified by Stewart et.al. (1985), were introduced into the experimental flock 49 days prior to the first vaccination. The sheep in groups 1 and 3 received one 2 ml dose of vaccine subcutaneously in the neck region, groups 2, 4, 5, 6 and 7 received two doses on opposite sides of the neck and group 8 was not vaccinated. The second dose of vaccine was administered to the appropriate group 35 days after the first. Sheep were bled 49 days prior to vaccination (at the time of introduction of donors), at the first and second vaccination and 28 days after the second (63 days after the first). The feet of all sheep were inspected at -49, 0, 21, 35 and 63 days after the first vaccination and the lesions evaluated by the scoring system of Stewart et.al. (1982, 1983b).

A. Vaccines

1. Strain 198 (serogroup AI) recombinant pili 50 μg per dose - one dose.
2. Strain 198 recombinant pili 50 μg per dose - two doses.
3. Strain 198 recombinant pili 12.5 μg per dose - one dose.
4. Strain 198 recombinant pili 12.5 μg per dose - two doses.
5. Strain 334 (serogroup B2) 50 fold concentrated culture supernatant (crude protease) - two doses.
6. Strain 198 basic protease 50 μg per dose, produced as described for Example 1 - two doses.
7. Strain 198 purified protease isoenzymes V1, V2, V3, V5, produced as described in Australian Patent Application No. 51387/85- two doses.
8. Unvaccinated controls.

The basic protease and isoenzymes were purified from B.nodosus strain 198 grown in Trypticase-arginine-serine (TAS) broth containing 0.15% $CaCl_2$. The crude protease vaccine was prepared from a highly proteolytic virulent strain (334) in a serogroup (B2) heterologous to the challenge strain (198,AI). Strain 334 was also grown in TAS broth medium containing 0.15% $CaCl_2$. After removal of the strain 334 cells by centrifugation the supernatant was concentrated 50 fold on a Diaflo YMI0 ultrafilter membrane in a Model 8400 stirred cell (Amicon). The amount of basic protease in vaccine 5 was estimated by a sandwich ELISA as described in Example 4. Purified strain 198 basic protease was used as a standard. Recombinant pili were prepared from Pseudomonas aeruginosa PAO DB2 harbouring a chimeric plasmid derived from pKT231 and the pilin gene of B.nodosus strain 198 controlled by the strong prokaryotic $P_R$ promotor (Elleman et.al. 1986).

12

B. Results

The basic protease vaccine induced both a marked curative and protective effect against natural field challenge with the homologous strain of B.nodosus (Table 6). This effect was evident after both the first and second dose of vaccine with fewer feet affected with either interdigital skin or underrunning lesions than the controls. The vaccines prepared from either homologous protease isoenzymes or heterologous crude protease supernatant also exerted a protective curative effect. After the first dose the effect was less obvious than with the basic protease vaccine although there were fewer underrunning lesions than in the controls. After the second dose there was a marked reduction in both interdigital skin and underrunning lesions in comparison with the controls. The basic protease vaccine performed consistently better than either the 50 $\mu$g or 12.25 $\mu$g recombinant pili vaccines. After the second dose of vaccine, the groups of sheep immunised with either the heterologous crude protease or the homologous isoenzyme vaccine had fewer feet affected with underrunning lesions than any of the groups vaccinated with recombinant pili. These results were unexpected as it was anticipated that the protection achieved with the recombinant pili vaccines would have been greater than that actually obtained.

Samples of lesion material from vaccinated and control sheep were cultured and the serogroup identity of the freshly isolated strains of B.nodosus was checked with typing antisera in agglutination tests (Claxton et.al. 1983). The virulence characteristics of the fresh isolates were checked by colony morphology, elastase and zymogram tests (Stewart et.al. 1986c). The results of these tests confirmed that several virulent strains belonging to 3 different serogroups (AI, BI and I) were present in the flock. This finding provides an explanation for the much better performance of the protease vaccines which induced cross-protective immunity against the heterologous serogroups. By comparison, recombinant pili vaccines which induce serogroup-specific immunity comparable to that induced by native pili (Elleman et.al. 1986; Stewart and Elleman 1987) were much less effective against these heterologous strains.

TABLE 6 Results of a field experiment showing percentage of feet affected with footrot in sheep challenged with strain 198 49 days prior to first vaccination.

| Vaccine group | %feet affected | | | | | | | | Body Weight loss(kg) |
|---|---|---|---|---|---|---|---|---|---|
| | Day 0* 2-4 (a) | (b) $\geqslant$3c | Day 21 2-4 | $\geqslant$3c | Day 35# 2-4 | $\geqslant$3c | Day 63 2-4 | $\geqslant$3c | |
| 1. Strain 198 recombinant pili (1 dose) 50 µg | 43.3 | 3.3 | 25.0 | 0 | 36.7 | 11.7 | 45.0 | 30.0 | -5.7 |
| 2. Strain 198 recombinant pili (2 doses) 50 µg | 25.0 | 0 | 33.3 | 0 | 35.0 | 21.7 | 40.0 | 18.3 | -3.9 |
| 3. Strain 198 recombinant pili (1 dose) 12.5µg | 38.3 | 1.7 | 28.3 | 5 | 35.0 | 18.3 | 41.7 | 18.3 | -5.53 |
| 4. Strain 198 recombinant pili (2 doses) 12.5 µg | 36.7 | 8.3 | 41.6 | 11.7 | 45.0 | 16.7 | 45.0 | 23.3 | -7.17 |
| 5. Strain 334 crude protease (2 doses) | 30.0 | 1.7 | 51.7 | 10 | 50 | 11.7 | 23.3 | 1.7 | -2.36 |
| 6. Strain 198 basic protease (2 doses) 50 µg | 38.3 | 3.3 | 38.3 | 13.3 | 20 | 6.7 | 13.3 | 3.3 | -2.06 |
| 7. Strain 198 protease isoenzymes (2 doses) 50 µg | 38.3 | 3.3 | 76.7 | 13.3 | 63.3 | 23.3 | 35.0 | 1.7 | -2.03 |

```
8. Unvaccinated
   controls   38.3  6.7 63.3  23.3 65.0 45.0 50.0 38.3 -8.24
```

```
*    first dose of vaccine
#    second dose of vaccine
(a)  all categories of footrot
(b)  severe underrunning lesions.
```

All of the groups vaccinated with recombinant pili had markedly elevated pilus agglutinating antibody titres to strain 198 whereas those vaccinated with the protease vaccine were similar to the controls (Table 7).

TABLE 7

| Mean strain 198 pilus agglutinating antibody titres of vaccinated groups of sheep in a field experiment. | | | |
|---|---|---|---|
| Vaccine group | Agglutinin titres (days after vaccination) | | |
| | 0 | 35 | 63 |
| 1. Strain 198 recombinant pili (one dose) 50 $\mu$g | <44 | 20,319 | 48,887 |
| 2. Strain 198 recombinant pili (two doses) 50 $\mu$g | <40 | 29,407 | 162,549 |
| 3. Strain 198 recombinant pili (one dose) 12.5 $\mu$g | <35 | 19,401 | 33,779 |
| 4. Strain 198 recombinant pili (two doses) 12.5 $\mu$g | <50 | 21,280 | 56,529 |
| 5. Strain 334 crude protease (two doses) | <38 | 260 | 133 |
| 6. Strain 198 basic protease (two doses) 50 $\mu$g | <29 | 188 | 127 |
| 7. Strain 198 protease isoenzymes (two doses) 50 $\mu$g | <32 | 260 | 226 |
| 8. Unvaccinated controls | <38 | 351 | 266 |

TABLE 8

| Mean ELISA antibody titres to the strain 198 basic protease in vaccinated and control sheep in a field experiment. | | | | |
|---|---|---|---|---|
| Vaccine group | ELISA antibody titres[1] days after vaccination | | | |
| | -49 | 0* | 35 + | 63 |
| 1. S198 recombinant pili (1 dose) | <5§ | <5§ | ND | 101# |
| 2. S198 recombinant pili (2 doses) | 17.7§ | 14§ | ND | 72# |
| 3. S198 recombinant pili (1 dose) | <5§ | <5§ | ND | 64# |
| 4. S198 recombinant pili (2 doses) | <5§ | <5§ | ND | 62# |
| 5. S334 crude protease (2 doses) | <5§ | <5§ | 1,163# | 2,213# |
| 6. S198 basic protease (2 doses) | <5§ | <5§ | 11,021# | 22,110# |
| 7. S198 protease isoenzyme (2 doses) | <5§ | <5§ | 2,381# | 2,917# |
| 8. Unvaccinated controls | <5# | <5# | 192# | 314# |

[1] cut-off at $OD_{450} = 0.5$

* first dose of vaccine

+ second dose of vaccine

# geometric means of 15 sheep per group

§ geometric means of at least 4 sheep per group

ND not done.

The best antibody response to the basic protease was obtained in the group vaccinated with the same antigen although the group immunised with strain 334 crude protease and strain 198 isoenzymes also had elevated mean titres (Table 8). A significant but smaller increase in mean titre was also observed in the control group and in these vaccinated with recombinant pili due to antibody induced by the presence of severe chronic infection.

EXAMPLE 3

The experiment was conducted on irrigated pasture at the CSIRO Werribee Field Station utilizing flood irrigation to provide sufficient moisture for pasture growth and transmission of footrot. One hundred and thirty-five mature Merino wethers and ewes were ranked on body weight and randomly distributed to 9 equal groups of 15 (11 wethers and 4 ewes). Four donors infected with pure cultures of B.nodosus strain 198 (serogroup AI) by the method of Egerton and Roberts (1971) as modified by Stewart et.al. (1985), were introduced into the experimental flock 84 days prior to the first vaccination. The sheep in group 1 received one 2ml dose of vaccine subcutaneously in the neck region, groups 2, 3, 4, 5, 6, 7 and 8 received two doses on opposite sides of the neck and group 9 was not vaccinated. The second dose of vaccine was administered to the appropriate group 27 days after the first. Sheep were bled at the first and second vaccination and 35 days after the second (62 days after the first). The feet of all sheep were inspected at -84, 0, 27 and 62 days after the second vaccination and the lesions evaluated by the scoring system of Stewart et.al. (1982, 1983b).

A. Vaccines.

1. Strain 198 (serogroup AI) recombinant pili 50μg per dose - one dose.
2. Strain 198 recombinant pili 50μg per dose - two doses.
3. Strain 183 (serogroup B2) recombinant pili 50μg per dose - two doses.
4. Strain 334 (serogroup B2) 50 fold concentrated culture supernatant (crude protease) 23μg per dose - two doses.
5. Strain 334 basic protease 50μg per dose, produced as described for Example 1 - two doses.
6. Strain 198 basic protease 50μg per dose produced as described for Example 1 - two doses.
7. Strain 198 basic protease 50μg per dose, treated with PMSF.
8. Strain 198 purified protease isoenzymes V1, V2, V3, V5, 50μg per dose, produced as described in Australian Patent Application No. 51387/85 - two doses.
9. Unvaccinated controls.

The strain 198 and 334 basic protease and strain 198 isoenzymes were purified from the respective B.nodosus strains grown in Trypticase-arginine-serine (TAS) broth containing 0.15% $CaCl_2$. The crude protease vaccine was prepared from a highly proteolytic virulent strain (334) in a srogroup (B2) heterologous to the challenge strain (198,AI). Strain 334 was also grown in TAS broth medium containing 0.15% $CaCl_2$. After removal of the strain 334 cells by centrifugation the supernatant was concentrated 50-fold on a Diaflo spiral-wound ultrafiltration membrane cartridge Model SIYl0 (Amicon). The amount of basic protease in vaccine 4 was estimated by a sandwich ELISA described in Example 4. Purified strain 198 basic protease was used as a standard. Strains 198 and 183 recombinant pili were prepared from Pseudomonas aeuruginosa PAO DB2 harbouring a chimeric plasmid derived from pKT231 and the pilin genes of B.nodosus strain 198 and 183 controlled by the strong prokaryotic $P_R$ promoter (Elleman et.al. 1986).

As in Example 2, the untreated and PMSF treated basic protease vaccines exerted a curative and protective effect against footrot with the homologous strain using natural field transmission (Table 9). The heterologous (strain 334) basic protease vaccine was as effective as the untreated strain 198 basic protease vaccine. The best results were obtained with PMSF treated strain 198 basic protease showing that inactivation of the serine residue in the active site of the protease does not destroy the protective epitope but in fact may enhance the immunogenic properties of the basic protease. The homologous recombinant pili vaccines induced serogroup specific immunity as shown previously (Elleman et.al. 1986; Stewart and Elleman 1987). By comparison, the heterologous recombinant pili vaccine (strain 183) was ineffective. The vaccines prepared from either homologous protease isoenzymes or heterologous crude protease supernatant also exerted a protective curative effect.

Samples of lesion material from vaccinated and control sheep were cultured on 6 separate occasions spanning the entire experiment and the serogroup identity of the freshly isolated strains of B.nodosus was checked with typing antisera in agglutination tests (Claxton et.al. 1983). The virulence characteristics of the fresh isolates were checked by colony morphology, elastase, proteinase thermostability and zymogram tests (Stewart et.al. 1986c). The results of these tests on 24 different swabs confirmed that virulent strain 198 belonging to serogroup AI was present in the flock.

TABLE 9   Results of a field experiment showing percentage of feet affected with footrot in sheep challenged with strain 198, 84 days prior to first vaccination.

| Vaccine group | % feet affected | | | | | | Body wt. gain/loss (kg) |
|---|---|---|---|---|---|---|---|
| | Day 0* | | Day 27# | | Day 62 | | |
| | 2-4 (a) | ≥3c (b) | 2-4 | ≥3c | 2-4 | ≥3c | |
| 1. Strain 198 recombinant pili (1 dose) (50µg | 30 | 6.7 | 5 | 5 | 5 | 1.7 | +7.2 |
| 2. Strain 198 recombinant pili (2 doses) 50µg | 35 | 18.3 | 10 | 3.3 | 8.3 | 1.7 | +6.2 |
| 3. Strain 183 recombinant pili (2 doses) 12.5µg | 26.7 | 5 | 43.3 | 11.7 | 38.3 | 26.7 | -0.6 |
| 4. Strain 334 crude protease (2 doses) 23 µg | 31.7 | 6.7 | 36.7 | 5 | 28.3 | 3.3 | +3.6 |
| 5. Strain 334 basic protease (2 doses) 50µg | 40 | 25 | 28.3 | 8.3 | 25 | 11.7 | +3.9 |
| 6. Strain 198 basic protease (2 doses) 50µg | 46.7 | 13.3 | 45 | 11.7 | 31.7 | 8.3 | +1.8 |
| 7. Strain 198 basic protease | | | | | | | |

```
        treated PMSF
        (2 doses)
        50µg            41.7 13.3  23.3    6.7  20      1.7          +4.6

    8.  Strain 198
        protease
        isoenzymes
        (2 doses)
        50µg            30    5    41.7    6.7  18.3    5            +3.1

    9.  Unvaccinated
        controls.       55   16.7  60     31.7 48.3   43.3          -1.3
```

|   |   |
|---|---|
| * | first dose of vaccine |
| # | second dose of vaccine |
| (a) | all categories of footrot |
| (b) | severe underrunning lesions |

The two groups vaccinated with recombinant pili strain 198 and 183 had markedly elevated pilus aggulutinating antibody titres to the homologous organism whereas those vaccinated with the protease vaccine were similar to the controls (Table 10). The group vaccinated with strain 334 crude protease also had an increase in mean titre to strains 183 and 334 (both serogroup B2 strains).

TABLE 10 Mean pilus agglutinating antibody titres of vaccinated groups of sheep in a field experiment.

| Vaccine group | Agglutinin titres (days after vaccination) | | | | | |
|---|---|---|---|---|---|---|
| | 0 | 27 | | 62 | | |
| 1. Strain 198 recombinant pili (1 dose) 50μg | 96* | 12,222* | (15)+ | 24,444* | (16)+ | (76)# |
| 2. Strain 198 recombinant pili (2 doses) 50μg | 121 | 13,405 | (12) | 48,888 | (18) | (80) |
| 3. Strain 183 recombinant pili (2 doses) 50μg | 88 | 279(9,700) | | 232 | (26811) | (51,200) |
| 4. Strain 334 crude protease (2 doses) | 96 | 184 | (200) | 153 | (219) | (665) |
| 5. Strain 334 basic protease | 111 | 121 | (11) | 92 | (11) | (58) |
| 6. Strain 198 basic protease (2 doses) 50μg | 133 | 184 | (13) | 140 | (15) | (64) |
| 7. Strain 198 basic protease treated PMSF (2 doses) 50μg | 121 | 106 | (13) | 80 | (13) | (61) |
| 8. Strain 198 protease isoenzymes (2 doses) 50μg | 88 | 160 | (15) | 121 | (15) | (73) |
| 9. Unvaccinated controls | 127 | 168 | (12) | 168 | (12) | (55) |

```
*        Strain 198 geometric mean antibody titres
+        Strain 183 geometric mean antibody titres
#        Strain 334 geometric mean antibody titres.
```

All of the groups vaccinated with basic proteases, crude protease and isoenzymes had markedly elevated mean titres to the basic protease 62 days after vaccination in comparison to unvaccinated controls or those vaccinated with recombinant pili (Table 11). However, the latter groups had increases in mean titres at the end of the experiment when compared to mean titres 62 days previously. This was due to antibody induced by the chronic disease process in these groups of sheep.

The mean titre for the group of sheep vaccinated with protease isoenzymes was lower than that of the group vaccinated with crude protease which in turn was lower than that of sheep vaccinated with basic protease. The group of sheep immunised with PMSF treated strain 198 basic protease had the highest mean titre indicating that inactivation of the serine residue may be a means of improving the immunogenicity of the basic protease.

TABLE 11

| Mean ELISA antibody titres to the basic strain 198 protease in vaccinated and control sheep in a field experiment, | | |
|---|---|---|
| Vaccine group | ELISA antibody titres[1] days after vaccination | |
| | 0 | 62 |
| 1. Strain 198 recombinant pili (1 dose) | <12.7 + | 52* |
| 2. Strain 198 recombinant pili (2 doses) | 11.5* | 55* |
| 3. Strain 183 recombinant pili (2 doses) | 3.2* | 93* |
| 4. Strain 334 crude protease (2 doses) | < 5 + | 2,111* |
| 5. Strain 334 basic protease (2 doses) | < 8.8 + | 5,703* (6,333)# |
| 6. Strain 198 basic protease (2 doses) | 5.3 + | 5,862* (9,850)# |
| 7. Strain 198 PMSF treated basic protease (2 doses) | 5.1 + | 8,294* |
| 8. Strain 198 protease isoenzymes (2 doses) | 7.6 + | 1,057* |
| 9. Unvaccinated controls | 9,5* | 215* |

[1] Cut-off at $OD_{450} = 0.5$
* Geometric mean antibody titres of 15 sheep/group.
+ Geometric mean antibody titres of 5 sheep
# Geometric mean antibody titres of 15 sheep/group against strain 334 basic protease.

The following Examples illustrate the use of the basic protease in diagnostic tests for detecting the presence of virulent and intermediate footrot and discriminating these clinical entities from benign footrot.

EXAMPLE 4

This experiment utilised a sandwich ELISA modified from that of the pilus quantitation method of Fahey et.al. (1983) to detect basic protease in either TAS broth cultures or in swabs containing exudate from footrot lesions. The swabs were taken on two separate occasions (34 days, Table 13 and 42 days, Table 14) in a pen experiment from B.nodosus lesions in groups of sheep which had been challenged separately with different isolates of B.nodosus. The different isolates were representative of virulent, intermediate and benign strains and had been previously characterised according to serogroup, elastase, proteinase thermostability and protease zymogram characteristics (for review of methods see Stewart et.al. 1986c). The swabs were extracted with 0.3ml 0.01 M Tris saline 5mM $CaCl_2$ buffer pH8.0 by forcing a suspension containing the cotton swab between two syringes connected by a double hubbed needle.

Microtitre trays (Nunc-Immuno Plate II) were coated overnight at room temperature with 100μl/well of a 1:1000 dilution in 0.02 M sodium carbonate/0.025 M sodium bicarbonate buffer pH9.6 of hyperimmune sheep polyclonal IgG antiserum to the basic protease. The plate was then blocked with 0.1% Coffee-mate (coffee whitener, Carnation) in 0.01 M PBS pH7.2, washed (0.01 M PBS pH7.2) and dilutions of either extracted lesion material (1:2, 1:5, 1:25) or broth culture (1:5, 1:25, 1:125, 1:625, etc.) were made in 1% Coffee-mate. After incubation for 1h or overnight and washing, $100\mu l$ mouse ascites fluid containing a mouse anti-strain 198 basic protease monoclonal antibody (mAb8) diluted 1:1000 in 0.1% Coffee-mate, was added to each well. The plates were then incubated for 1h and then washed. The amount of mouse Ig binding to the basic protease was then detected using horse radish peroxidase conjugated sheep anti-mouse Ig antiserum (Silenus) diluted 1:1000 in 0.1% Coffee-mate in PBS followed by washing and the addition of substrate (5-aminosalicylic acid/$H_2O_2$). After incubation for 1-2h the optical density of each well was read on an ELISA autoreader spectrophotometer (Bio-tek Instruments, Inc., Model EL310) at a wavelength of 450nm.

RESULTS

Broth cultures of virulent and intermediate strains contained much more basic protease as detected by the sandwich ELISA than benign strains (Table 12).

23 of the 24 swabs of exudate from lesions of virulent and intermediate footrot contained substantial amounts of basic protease in contrast to the negligible quantity in the swabs from the benign footrot lesion (Tables 13 and 14). Clear true positive results were obtained for the virulent strain 198 (lesion scores 1, 3a) and negative results were obtained for the benign strain 463 when lesions of similar severity (lesion scores 1, 3b) were tested.

TABLE 12 A sandwich ELISA diagnostic test for determination of virulence based on detection of the basic protease in B.nodosus TAS broth culture containing 0.15% $CaCl_2$

| Strain | Sero-group | Virulence | Cell count $(X10^8)$ | ELISA titre | ELISA titre per $10^8$ cells |
|--------|-----------|-----------|----------------------|-------------|------------------------------|
| **1.** | **Four day old cultures** | | | | |
| 198 | A1 | Virulent | 1.2 | 105 | 87 |
| 476 | A1 | Virulent | 1.0 | 105 | 105 |
| 394 | A1 | Benign | 1.6 | ‹ 5 | ‹ 5 |
| 466 | H1 | Intermediate | 2.0 | 53 | 26 |
| 265 | H1 | Virulent | 1.6 | 250 | 156 |
| 462 | A1 | Benign | 1.4 | 11 | 8 |
| 463 | A1 | Benign | 1.4 | 6.5 | 5 |
| **2.** | **Three day old cultures** | | | | |
| 408 | B2 | Intermediate | 2.0 | 115 | 57 |
| 337 | I | Benign | 1.0 | 5.5 | 5.5 |
| 476 | A1 | Virulent | 2.0 | 225 | 112 |
| 234 | B1 | Virulent | 2.0 | 110 | 55 |
| 469 | F1 | Virulent | 2.0 | 135 | 67 |
| **3.** | **Three day old cultures** | | | | |
| 338 | G1 | Virulent | 0.8 | 100 | 125 |
| 463 | A1 | Benign | 1.2 | 6 | 5 |
| 198 | A1 | Virulent | 1.2 | 260 | 217 |
| 480 | B1 | Benign | 0.8 | 5 | 6 |
| 481 | B1 | Benign | 1.4 | ‹5 | ‹5 |
| 246 | G1 | Virulent | 1.0 | 100 | 100 |
| 305 | C2 | Benign | 0.8 | 8 | 10 |

TABLE 13

| A sandwich ELISA diagnostic test for determination of virulence based on detection of the basic protease in lesion exudate from sheep affected with footrot. | | | | | | | |
|---|---|---|---|---|---|---|---|
| Sheep No. (foot) + | Strain | Sero-group | Virulence | Optical density (450nm) | | | |
| | | | | 1 hour* | | overnight* | |
| | | | | 1:2 | 1:5 | 1:2 | 1:5 |
| | | | | (dilution factor) | | (dilution factor) | |
| 646(LH) | 463 | AI | Benign | 0.08 | 0.08 | 0.14 | 0.14 |
| 763(LH) | 469 | FI | Virulent | 1.27 | 0.05 | 1.16 | 0.44 |
| 597(RF) | 198 | AI | Virulent | 0.69 | 0.29 | 1.14 | 0.63 |
| 574(LF) | 466 | HI | Intermed | 0.35 | 0.12 | 0.60 | 0.24 |
| 597(LH) | 198 | AI | Virulent | 0.51 | 0.13 | 1.21 | 0.33 |
| 597(RH) | 198 | AI | Virulent | 0.05 | 0.06 | 0.21 | 0.13 |

* Period of incubation of lesion material on plates coated with sheep polyclonal antiserum to the basic protease.
 + The foot lesion swabbed is identified as left front (LF), right front (RF), left hind (LH), right hind (RH).

TABLE 14 A sandwich ELISA diagnostic test for determination of virulence based on detection of the basic protease in lesion exudate from sheep affected with footrot.

| Sheep No. (foot)# | Strain | Sero- Group | Virulence | Lesion Score+ | Optical density (450nm) 1 hour * 1:2 | 1:5 (dilution factor) |
|---|---|---|---|---|---|---|
| 583(RH) | 408 | B2 | Intermediate | 4b | 0.86 | 0.38 |
| 701(LF) | 408 | B2 | Intermediate | 3a | 0.33 | 0.19 |
| 781(LF) | 408 | B2 | Intermediate | 4a | 0.78 | 0.35 |
| 829(RF) | 408 | B2 | Intermediate | 3b | 0.46 | 0.15 |
| 506(RF) | 450 | A1 | Intermediate | 4b | 0.58 | 0.36 |
| 626(LF) | 450 | A1 | Intermediate | 4a | 1.08 | 0.56 |
| 950(LH) | 463 | A1 | Benign | 1 | 0.07 | 0.06 |
| 981(RH) | 463 | A1 | Benign | 3b | 0.15 | 0.07 |
| 436(LF) | 198 | A1 | Virulent | 4b | 1.75 | 1.42 |
| 606(RF) | 198 | A1 | Virulent | 4b | 0.56 | 0.35 |
| 771(RH) | 198 | A1 | Virulent | 4b | 0.95 | 0.30 |
| 897(RF) | 198 | A1 | Virulent | 4b | 1.19 | 1.36 |
| 897(RH) | 198 | A1 | Virulent | 46 | 0.99 | 0.46 |
| 906(RF) | 198 | A1 | Virulent | 4b | 1.21 | 0.51 |
| 764(Front) | 469 | F1 | Virulent | 4b | 0.45 | 0.31 |
| 485(LF) | 466 | H1 | Intermediate | 4a | 1.18 | 0.77 |
| 722(LH) | 466 | H1 | Intermediate | 4a | 0.31 | 0.15 |
| 461(RF) | 198 | A1 | Virulent | 3a | 0.78 | 0.51 |
| 465(LH) | 198 | A1 | Virulent | 1 | 1.11 | 0.69 |
| 893(LH) | 198 | A1 | Virulent | 3a | 1.53 | 1.00 |
| 430(LF) | 198 | A1 | Virulent | 2 | 1.29 | 0.75 |

```
*        Period of incubation of lesion material on plates
         coated with sheep polyclonal antiserum to the basic
         protease.
+        The severity of thelesion of footrot using the
         scoring system of Stewart et.al. (1982, 1983b).
#        The foot lesion swabbed is identified as left front
         (LF), right front (RF), left hind (LH), right hind
         (RH).
```

EXAMPLE 5

Antibody induced in response to infection was detected in the serum of unvaccinated sheep infected with virulent B.nodosus. Microtitre plates (Nunc-Immuno Plate II) were coated with mouse ascites containing mAb8 (see Example 4) diluted 1:1000 in sodium carbonate/bicarbonate buffer pH9.6. The plates were blocked with 0.1% Coffee-mate, washed and 10ng/100$\mu$l strain 198 basic protease in 0.1% Coffee-mate in PBS, was added to each well. Serial dilutions of the test antiserum in 1% Coffee-mate in PBS were transferred to the wells. After incubation for 1h and washing a 1:1000 dilution of HRP donkey antisheep Ig antiserum (Silenus) in 0.1% Coffee Mate in PBS was added to each well. After further incubation, washing and addition of substrate (5AS/$H_2O_2$) the optical density of each well was read at 450nm.

RESULTS

The two most virulent strains (469 and 198) induced a substantially greater antibody response than anyof the other strains and this was evident 39-42 days after challenge (Table 15). Seven out of 8 sheep (titre range 65-650) and 8 out of 8 sheep (titre range 78-1,650) in the strain 469 and strain 198 challenge groups, respectively developed a marked increase in antibody titre after challenge when compared to pre-infection antibody levels.

By contrast only 2 sheep in each group of 8 challenged with the intermediate strains 466, 408 and 450 developed antibody titres greater than 50 (titres 83 and 93, 50 and 475, 104 and 275 respectively). None of the sheep challenged with the benign strain 463 developed titres above 40 with 6 out of 8 sheep having titres <25. Thus the ELISA serum antibody test using the basic protease of B.nodosus may be useful for screening flocks for the presence of virulent footrot.

TABLE 15

| Relationship between virulence of the challenge strain, severity of footrot and mean ELISA serum antibody titre to the basic protease in groups of 8 sheep per strain challeng group. | | | | | | | | |
|---|---|---|---|---|---|---|---|---|
| Strain | Sero- | Virulence | % feet with lesions of footrot | | | | Mean ELISA anti-body titre* | |
| | | | 3 weeks | | 6 weeks | | Pre- | Post- |
| | | | ≥3c | ≥2 | ≥3c | ≥2 | Infection | |
| 469 | Fl | Virulent | 37.5 | 100 | 100 | 100 | 13 | 154 + |
| 466 | Hl | Intermed. | 12.5 | 59.4 | 59.4 | 78.1 | 10 | 17 + |
| 408 | B2 | Intermed. | 6.3 | 87.5 | 56.3 | 93.8 | 10 | 23# |
| 450 | Al | Intermed. | 9.4 | 90.6 | 89.3 | 100 | 10 | 20# |
| 463 | Al | Benign | 0 | 12.15 | 0 | 3.1 | 9 | 13# |
| 198 | Al | Virulent | 75 | 100 | 100 | 100 | 10 | 525# |

* Cut-off at $OD_{450} = 0.35$

+ Geometric mean antibody titre 42 days after challenge Geometric mean antibody titre 39 days after challenge.

CONCLUSION

Examples 1, 2 and 3 demonstrate that protection can be induced by the basic protease against a highly virulent strain of the homologous serogroup (A). In addition, Examples 2 and 3 demonstrate that the basic protease induces cross-protection against 2 serogroups (B and I) distinct from each other and the serogroup (A) from which the antigen was prepared. This suggests that the basic protease has common antigens or serological determinants which are shared by at least some serogroups.

The basic protease of B.nodosus is thus a promising cross-protective antigen for providing broad-spectrum cover against a wide range of serogroups. The present invention extends to the use for vaccination against footrot of the basic protease produced either by B.nodosus itself or through recombinant DNA technology which allows the transfer of the genes encoding the basic protease of B.nodosus into a new host. The new host in which the protease antigen is produced from cloned B.nodosus gene sequences may be another bacterium such as species selected from Bacillus, Escherichia and Pseudomonas or any other type of cell in which the genes are capable of being expressed (i.e. translated into the protein antigen product) with or without genetic modification of the host genes or additional genetic engineering of the cloned B.nodosus gene sequences themselves. Thus cloning of the basic protease by recombinant DNA technology may provide the solution to the problem of producing large amounts of this antigen required for vaccine manufacture, and the present invention extends to the use of the basic protease produced either by B.nodosus itself or by means of cloning of the antigen into a new host.

It is well known that it is often not necessary to use a whole protein antigen to generate antibodies active against the protein or the corresponding infectious agent. It is sometimes possible to use polypeptide fragments for this purpose. It is therefore within the ambit of this invention to use as the protease antigen related immunogenic fragments derived from modified B.nodosus basic protease genes, related protease genes or fragments expressed in the new host or chemically synthesized peptides. The term "basic protease" as used throughout this specification and claims is therefore to be understood as extending to include such immunogenic fragments.

It will be appreciated by those skilled in the art that the proteases produced by B.nodosus offer several advantages over other antigens for inducing protection, particularly the pili antigens. In particular:

(a) the proteases from the different serogroups so far investigated have common antigenic determinants,

(b) the proteases are excreted by B.nodosus outside the cell into the culture medium, and

(c) very high levels of protease production from cloned genes may be achieved by genetic engineering techniques in host organisms with deletion or insertion mutations in their endogenous protease genes.

Examples 5 and 6 demonstrate that the basic protease of B.nodosus can be used in diagnostic tests to discriminate virulent and intermediate strains of B.nodosus from benign strains. Since the basic protease produced by virulent strains can be detected in swabs of lesion exudate, this provides a promising basis for a rapid diagnostic test. There is also provided a method of detecting antibodies in sheep serum, resulting from infection of sheep's feet with strains of B.nodosus causing virulent and intermediate footrot, which comprises the use of the basic protease of B.nodosus as an antigen in an immunoassay. Thus the present invention extends to diagnostic tests to detect the presence of such virulent or intermediate strains both in vivo and in vitro. Such diagnostic tests may be based on the use of monoclonal antibodies produced to the basic protease, or on the use of oligonucleotide, DNA or RNA probes corresponding to unique basic protease gene sequences.

REFERENCES

1. Claxton, P.D., Ribeiro, L.A. and Egerton, J.R. Classification of Bacteroides nodosus by agglutination tests. Australian Veterinary Journal, (1983), 60 : 331-334.

2. Davis, J.B. Disc electrophoresis.II. Method and application to human serum proteins. Annals of the New York Academy of Sciences (1986) 121 : 404-427.

3. du Cros, D.L. and Wrigley, C.W. Improved electrophoretic methods for identifying cereal varieties. Journal of the Science of Food and Agriculture (1979), 30 : 785-794.

4. Egerton, J.R. and Roberts, D.S. Vaccination against ovine footrot. Journal of Comparative Pathology - (1971), 81 : 179-185.

5. Elleman, T.C., Hoyne, P.A., Emery, D.L., Stewart, D.J. and Clark, B.L. Isolation of the gene encoding pilin of Bacteroides nodosus (Strain 198), the causal organism of ovine footrot. FEBS Letters, (1984), 173 : 103-107.

6. Elleman, T.C. and Hoyne, P.A. Nucleotide sequence of the gene encoding pilin of Bacteroides nodosus, the causal organism of ovine footrot. Journal of Bacteriology (1984), 160 : 1184-1187.

7. Elleman, T.C., Hoyne, P.A., Stewart, D.J., McKern, N.M. and Peterson, J.E. Expression of pili from Bacteroides nodosus in Pseudomonas aeruginosa. Journal of Bacteriology (1986), 168 : 574-580.

8. Every, D. Proteinase isoenzyme patterns of Bacteroides nodosus : Distinction between ovine virulent isolates, ovine benign isolates and bovine isolates. Journal of General Microbiology, (1982), 128 : 809-812.

9. Every, D. and Skerman, T.M. Protection of sheep against experimental footrot by vaccination with pili purified from Bacteroides nodosus. New Zealand Veterinary Journal, (1982), 30 : 156-158.

10. Fahey, K.J., McWaters, P.G., Stewart, D.J., Peterson, J.E. and Clark, B.L. Quantitation by ELISA of pili and sheep antibodies to the pili of Bacteroides nodosus. Australian Veterinary Journal, (1983), 60:111-116.

11. Hartree, E.F. Determination of Protein : A modification of the Lowry Method that gives a linear photometric response. Analytical Biochemistry, (1972), 48 : 422-427.

12. Kortt, A.A., Burns, J.E. and Stewart, D.J. Detection of the extracellular proteases of Bacteroides nodosus in polyacrylamide gels: a rapid method of distinguishing virulent and benign ovine isolates. Research in Veterinary Science, (1983), 35 : 171-174.

13. Kortt, A.A., Caldwell, J.B., Burns, J.E. Guthrie, R.E. and Stewart, D.J. Characterisation and substrate specificity of the extracellular proteases of Bacteroides nodosus from virulent and benign ovine isolates. In Footrot in Ruminants, Proceedings of a Workshop, Melbourne 1985. Edited by D.J.Stewart, J.E.Peterson, N.M. McKern and D.L.Emery. CSIRO Divison of Animal Health and Australian Wool Corporation, (1986), pp.237-243.

14. Kortt, A.A., O'Donnell, I.J., Stewart, D.J. and Clark, B.L. Activities and partial purification of extracellular proteases of Bacteroides nodosus from virulent and benign footrot. Australian Journal of Biological Sciences, (1982), 35 : 481-489.

15. Kortt, A.A., Strike, P.M., Bogusz, D. and Appleby, C.A. The amino acid sequence of leghemoglobin II from Sesbania rostrata stem nodules. Biochemistry International, (1987), 15:509-516.

16. Reisfeld, R.A., Lewis, U.J. and Williams, D.E. Disk electrophoresis of basic proteins and peptides on polyacrylamide gels. Nature (Lond.), (1962), 195 : 281-283.

17. Stewart, D.J. The role of various antigenic fractions of Bacteroides nodosus in eliciting protection against footrot in vaccinated sheep. Research in Veterinary Science, (1978), 24 : 14-19.

18. Stewart, D.J., Clark, B.L., Emery, D.L., Peterson, J.E. and Fahey, K.J. A Bacteroides nodosus immunogen, distinct from the pilus, which induces cross-protective immunity in sheep vaccinated against footrot. Australian Veterinary Journal, (1983a), 60 : 83-85.

19. Stewart, D.J., Clark, B.L., Emery, D.L., Peterson, J.E., Jarrett, R.G. and O'Donnell, I.J. Cross-protection from Bacteroides nodosus vaccines and the interaction of pili and adjuvants. Australian Veterinary Journal, (1986a), 63 : 101-106.

EP 0 362 274 B1

20. Stewart, D.J., Clark, B.L., Emery, D.L,, Peterson, J.E. and Kortt, A.A. The phenomenon of cross protection against footrot induced by vaccination of sheep with Bacteroides nodosus vaccines. In Footrot in Ruminants, Proceedings of a Workshop, Melbourne, 1985. Edited by D.J.Stewart, J.E.Peterson, N.M.McKern and D.L. Emery. CSIRO Division of Animal Health and Australian Wool Corporation (1986b), pp.185-192.

21. Stewart, D.J., Clark, B.L. and Jarrett, R.G. Observations on strains of Bacteroides nodosus of intermediate virulence to sheep. Australian Advances in Veterinary Science, (1982), pp.219-222.

22. Stewart, D.J., Clark, B.L., Peterson, J.E., Emery, D.L., Smith, E.F., Griffiths, D.A. and O'Donnell,I.J. The protection given by pilus and whole cell vaccines of Bacteroides nodosus strain 198 against ovine foot-rot induced by strains of different serogroups. Australian Veterinary Journal, (1985), 62 : 153-159.

23. Stewart, D.J., Clark, B.L., Peterson, J.E., Griffiths, D.A. and Smith, E.F. Importance of pilus-associated antigen in Bacteroides nodosus vaccines. Research in Veterinary Science, (1982), 32 : 140-147.

24. Stewart, D.J., Clark, B.L., Peterson, J.E., Griffiths, D.A., Smith, E.F. and O'Donnell,I.J. Effect of pilus dose and type of Freund's adjuvant on the antibody and protective responses of vaccinated sheep to Bacteroides nodosus. Research in Veterinary Science, (1983b), 35 : 130-137.

25. Stewart, D.J. and Elleman, T.C. A Bacteroides nodosus pili vaccine produced by recombinant DNA for the prevention and treatment of footrot in sheep. Australian Veterinary Journal, (1987), 64 : 79-81.

26. Stewart, D.J., Peterson, J.E., Vaughan, J.A., Clark, B.L., Emery, D.L., Caldwell, J.B. and Kortt, A.A. The pathogenicity and cultural characteristics of virulent intermediate and benign strains of Bacteroides nodosus causing ovine foot-rot. Australian Veterinary Journal (1986c), 63 : 317-326.

27. Thorley, C.M. and Egerton, J.R. Comparison of alum-absorbed or non-alum-absorbed oil emulsion vaccines containing either pilate or non-pilate Bacteroides nodosus cells in inducing and maintaining resistance of sheep to experimental foot-rot. Research in Veterinary Science, (1981), 30 : 32-37.

**Claims**

1. A vaccine for use in protection against or treatment of footrot, the vaccine containing an immunologically effective amount of the basic serine protease of Bacteroides nodosus as the active component thereof together with an acceptable pharmaceutical or veterinary diluent or carrier therefor, the basic serine protease having a pI of about 9.

2. A vaccine according to claim 1 further containing adjuvant.

3. A vaccine according to claim 2 wherein said adjuvant is an oil, aluminium hydroxide, saponin-aluminium hydroxide or aluminium hydroxide-oil adjuvant.

4. A vaccine according to claim 1 containing said basic protease in a form selected from the purified basic protease, culture supernatant of B.nodosus partially purified to provide an immunologically effective amount of the basic protease, and crude culture supernatant of B.nodosus concentrated to provide an immunologically effective amount of the basic protease.

5. A vaccine according to claim 1 containing a synthetic polypeptide displaying the antigenicity of the basic serine protease.

6. A vaccine according to claim 5 wherein said synthetic polypeptide comprises the expression product of a host cell containing a recombinant DNA cloning vehicle which comprises an expression control sequence and a nucleotide sequence capable of being expressed as all or at least a part of the basic serine protease of B.nodosus, or a peptide comprising at least one epitope thereof.

7. A vaccine according to claim 1 wherein said basic protease is derived from virulent strain 198 or virulent strain 334 of B.nodosus.

8. A recombinant DNA molecule comprising a nucleotide sequence capable of being expressed as all or at least a part of the basic (pI about 9) serine protease of B.nodosus, or a peptide comprising at least one epitope thereof.

9. A recombinant DNA molecule according to claim 8 comprising a nucleotide sequence substantially as set out in Table 2.

**10.** A recombinant DNA cloning vehicle comprising an expression control sequence and a nucleotide sequence capable of being expressed as a polypeptide having the antigenicity of the basic (pI about 9) serine protease of B.nodosus.

**11.** A host cell containing a recombinant DNA cloning vehicle according to claim 10.

**12.** A synthetic polypeptide displaying the antigenicity of the basic (pI about 9) serine protease of B.nodosus.

**13.** A synthetic peptide according to claim 12 comprising an amino acid sequence substantially as set out in Table 2.

**14.** An antibody to the basic (pI about 9) serine protease of B.nodosus.

**15.** An antibody according to claim 14 which is a monoclonal antibody.

**16.** An antibody according to claim 15 which is a mouse, ovine or bovine monoclonal antibody.

**17.** A diagnostic test for the detection of the presence of virulent and/or intermediate strains of B.nodosus in a sample, which is characterised by the use of at least one antibody according to claim 14 in an immunoassay.

**18.** A diagnostic test according to claim 17 which comprises the steps of :
   [a] contacting the sample with a support having immobilised thereon an antibody according to claim 14; and
   [b] detecting the presence of binding of said basic serine protease of virulent and/or intermediate strains to said antibody.

**19.** A diagnostic test according to claim 18 wherein said step [b] comprises addition of a monoclonal antibody to the basic serine protease, followed by detection of bound monoclonal antibody.

**20.** A diagnostic test kit for the detection of the presence of virulent and/or intermediate strains of B.nodosus in a sample, which comprises :
   [a] a support having immobilised thereon an antibody according to claim 14; and
   [b] means for detecting the binding of said basic serine protease of virulent and/or intermediate strains to said antibody.

**21.** A diagnostic test for the detection of antibodies to B.nodosus in a serum sample, which comprises the use of the basic (pI about 9) serine protease of B.nodosus in an immunoassay.

**22.** A diagnostic test according to claim 21 which comprises the steps of :
   [a] contacting the serum sample with a support having the basic (pI about 9) serine protease of B.nodosus immbolilsed thereon; and
   [b] detecting the presence of antibodies in the serum sample bound to the support.

**23.** A diagnostic test kit for the detection of antibodies to B.nodosus in a serum sample, which comprises :
   [a] a support having the basic (pI about 9) serine protease of B.nodosus immobilised thereon; and
   [b] means for detecting the presence of antibodies in the serum sample bound to the support.

**Patentansprüche**

**1.** Impfstoff zum Schutz gegen bzw. für die Behandlung von Moderhinke mit einer immunologisch wirksamen Menge der basischen Serinprotease von Bacteroides nodosus als Aktivbestandteil, zusammen mit einem pharmazeutisch oder veterinärmedizinisch annehmbaren Verdünnungsmittel oder Träger dafür, wobei der pI der basischen Serinprotease ungefähr 9 beträgt.

**2.** Impfstoff nach Anspruch 1, der weiterhin Hilfsstoff enthält.

**3.** Impfstoff nach Anspruch 2, wobei es sich bei dem Hilfsstoff um ein öl oder um Aluminiumhydroxid, Saponin-Aluminiumhydroxid oder Aluminiumhydroxidöl handelt.

**4.** Impfstoff nach Anspruch 1 mit der basischen Protease in einer der folgenden Formen: gereinigte basische Protease, Kulturüberstand von B. nodosus, der zum Erhalt einer immunologisch wirksamen Menge der basischen Protease teilgereinigt wurde, sowie Rohkulturüberstand von B. nodosus, der zum Erhalt einer immunologisch wirksamen Menge der basischen Protease konzentriert wurde.

**5.** Impfstoff nach Anspruch 1 mit einem synthetischen Polypeptid, das über die Antigenwirkung der basischen Serinprotease verfügt.

**6.** Impfstoff nach Anspruch 5, wobei das synthetische Polypeptid das Expressionsprodukt einer Wirtszelle enthält, das einen rekombinanten DNA-Klonierungsvektor mit einer Expressionskontrollsequenz und einer Nukleotidsequenz, die als vollständige bzw. zumindest teilweise vorhandene basische Serinprotease von B. nodosus exprimierbar sind, oder einem Peptid mit zumindest einem ihrer Epitope umfaßt.

**7.** Impfstoff nach Anspruch 1, wobei die basische Protease vom virulenten Stamm 198 oder dem virulenten Stamm 334 von B. nodosus stammt.

**8.** Rekombinantes DNA-Molekül mit einer Nukleotidsequenz, die als vollständige oder zumindest teilweise vorhandene basische (pI ungefähr 9) Serinprotease von B. nodosus exprimierbar, oder einem Peptid mit zumindest einem ihrer Epitop ist.

**9.** Rekombinantes DNA-Molekül nach Anspruch 8 mit einer im wesentlichen wie in Tabelle 2 dargestellten Nukleotidsequenz.

**10.** Rekombinanter DNA-Klonierungsvektor mit einer Expressionskontrollsequenz sowie einer Nukleotidsequenz, die als Polypeptid mit der Antigenwirkung der basischen (PI ungefähr 9) Serinprotease von B. nodosus experimierbar sind.

**11.** Wirtszelle mit einem rekombinanten DNA-Klonierungsvektor nach Anspruch 10.

**12.** Synthetisches Polypeptid mit der Antigenwirkung der basischen (pI ungefähr 9) Serinprotease von B. nodosus.

**13.** Synthetisches Peptid nach Anspruch 12 mit einer im wesentlichen wie in Tabelle 2 dargestellten Aminosäuresequenz.

**14.** Antikörper gegen die basische (PI ungefähr 9) Serinprotease von B. nodosus.

**15.** Antikörper nach Anspruch 14, wobei es sich um einen monoklonalen Antikörper handelt.

**16.** Antikörper nach Anspruch 15, wobei es sich um einen monoklonalen Maus-, Schaf- oder Rinderantikörper handelt.

**17.** Diagnoseverfahren zum Nachweis von virulenten und/oder intermediären Stämmen von B. nodosus in einer Probe, dadurch gekennzeichnet, daß man zumindest einen Antikörper nach Anspruch 14 in einem Immuntest verwendet.

**18.** Diagnosevervahren nach Anspruch 17, bestehend aus den folgenden Schritten:
[a] die Probe wird mit einem Träger in Kontakt gebracht, auf dem ein Antikörper nach Anspruch 14 immobilisiert ist; und
[b] die Bindung der basischen Serinprotease von virulenten und/oder intermediären Stämmen an den Antikörper wird nachgewiesen.

**19.** Diagnoseverfahren nach Anspruch 18, wobei man in Schritt [b] der basischen Serinprotease einen monoklonalen Antikörper zusetzt und anschließend die Bindung des monoklonalen Antikörpers nachweist.

**20.** Diagnosekit zum Nachweis von virulenten und/oder intermediären Stammen von B. nodosus in einer Probe, bestehend aus:
[a] einem Träger, auf dem ein Antikörper nach Anspruch 14 immobilisiert ist; und
[b] Mittel zum Nachweis der Bindung der basischen Serinprotease von virulenten und/oder intermediären Stämmen an den Antikörper.

**21.** Diagnoseverfahren zum Nachweis von Antikörpern gegen B. nodosus in einer Serumprobe, wobei man die basische (pI ungefähr 9) Serinprotease von B. nodosus in einem Immuntest verwendet.

**22.** Diagnoseverfahren nach Anspruch 21, bestehend aus den folgenden Schritten:
[a] die Serumprobe wird mit einem Träger in Kontakt gebracht, auf dem die basische (pI ungefähr 9) Serinprotease von B. nodosus immobilisiert ist; und
[b] Antikörper in der an den Träger gebundenen Serumprobe werden nachgewiesen.

**23.** Diagnosekit zum Nachweis von Antikörpern gegen B. nodosus in einer Serumprobe, bestehend aus:
[a] einem Träger, auf dem die basische (pI ungefähr 9) Serinprotease von B. nodosus immobilisiert ist; und
[b] Mittel zum Nachweis von Antikörpern in der an den Träger gebundenen Serumprobe.

**Revendications**

**1.** Vaccin destiné à être utilisé à des fins prophylactiques ou thérapeutiques vis-à-vis du fourchet, lequel vaccin contient une quantité efficace d'un point de vue immunologique de la sérine protéase basique de Bacteroides nodosus en tant que substance active accompagnée d'un diluant ou d'un porteur acceptable d'un point de vue pharmaceutique ou vétérinaire, le pI de la sérine protéase étant de 9 environ.

**2.** Vaccin selon la revendication 1 contenant en outre un adjuvant.

**3.** Vaccin selon la revendication 2, dans lequel ledit adjuvant est une huile, un hydroxyde d'aluminium, un hydroxyde d'aluminium-saponine ou un adjuvant huileux d'hydroxyde d'aluminium.

**4.** Vaccin selon la revendication 1 contenant ladite protéase basique sous une forme choisie parmi la protéase basique purifiée, un surnageant de culture de B. nodosus partiellement purifié de manière à fournir une quantité immunologiquement active de la protéase basique et un surnageant de culture de B. nodosus brut concentré de manière à fournir une quantité immunologiquement active de la protéase basique.

**5.** Vaccin selon la revendication 1 contenant un polypeptide synthétique présentant l'antigénicité de la sérine protéase basique.

**6.** Vaccin selon la revendication 5 dans lequel ledit polypeptide synthétique contient le produit d'expression d'une cellule hôte contenant un véhicule de clonage de l'ADN recombinant qui comprend une séquence régulatrice de l'expression et une séquence nucléotidique susceptible d'être exprimée sous forme de l'intégralité ou, au moins, d'une partie de la sérine protéase basique de B. nodosus. ou d'un peptide comprenant au moins un épitope de celle-ci.

**7.** Vaccin selon la revendication 1 dans lequel ladite protéase basique est issue de l'une des souches virulentes 198 ou 334 de B. nodosus.

**8.** Molécule d'ADN recombinant comprenant une séquence nucléotidique susceptible d'être exprimée sous forme de l'intégralité ou au moins d'une partie de la sérine protéase basique (pI égal 9 environ) de B. nodosus, ou d'un peptide comprenant au moins un épitope de celle-ci.

**9.** Molécule d'ADN recombinant selon la revendication 8 qui comprend une séquence nucléotidique substantiellement telle que définie dans le tableau 2.

31

**10.** Véhicule de clonage de l'ADN recombinant qui comprend une séquence régulatrice de l'expression et une séquence nucléotidique susceptible d'être exprimée sous forme d' un polypéptide ayant l' antigénicité de la sérine protéase basique (pI égal 9 environ) de B. nodosus.

**11.** Cellule hôte contenant un véhicule de clonage de l'ADN recombinant selon la revendication 10.

**12.** Peptide synthétique présentant l'antigénicité de la sérine protéase basique (pI égal 9 environ) de B. nodosus.

**13.** Peptide synthétique selon la revendication 12 comprenant une séquence d'acides aminés substantiellement telle que définie dans le tableau 2.

**14.** Anticorps dirigé contre la sérine protéase basique (pI égal 9 environ) de B. nodosus.

**15.** Anticorps selon la revendication 14 qui est un anticorps monoclonal.

**16.** Anticorps selon la revendication 15 qui est un anticorps monoclonal de souris, d'un ovin ou d'un bovin.

**17.** Test diagnostique destiné à la détection de la présence de souches virulentes et /ou intermédiaires de B. nodosus dans un échantillon, lequel est caractérisé par l'utilisation d'au moins un anticorps selon la revendication 14 dans un dosage immunologique.

**18.** Test diagnostique selon la revendication 17 qui comprend les étapes consistant à:
[a] placer l'échantillon au contact d'un support sur lequel est immobilisé un anticorps selon la revendication 14; et
[b] détecter la présence de la liaison de ladite sérine protéase basique de souches virulentes et/ou intermédiaires audit anticorps.

**19.** Test diagnostique selon la revendication 18 dans lequel ladite étape [b] comprend l'addition d'un anticorps monoclonal à la sérine protéase basique, suivie de la détection de l'anticorps monoclonal lié.

**20.** Nécessaire de test diagnostique destiné à la détection de la présence de souches virulentes et/ou intermédiaires de B. nodosus dans un échantillon, qui comprend :
[a] un support sur lequel est immobilisé un anticorps selon la revendication 14; et
[b] un moyen de détection de la liaison de ladite sérine protéase basique de souches vitulentes et/ou intermédiaires audit anticorps.

**21.** Test diagnostique destiné à la détection d'anticorps dirigés contre B. nodosus dans un échantillon de sérum, qui comprend l'utilisation de la sérine protéase basique (pI égal 9 environ) de B. nodosus dans un dosage immunologique.

**22.** Test diagnostique selon la revendication 21 qui comprend les étapes consistant à:
[a] placer l'échantillon au contact d'un support sur lequel est immobilisé la sérine protéase basique (pI égal 9 environ) de B. nodosus: et
[b] détecter la présence d'anticorps dans l'échantillon de sérum lié au support.

**23.** Nécessaire de test diagnostique destiné à la détection d'anticorps dirigés contre B. nodosus dans un échantillon de sérum, qui comprend:
[a] un support sur lequel est immobilisée la sérine protéase basique (pI égal 9 environ) de B. nodosus; et
[b] un moyen de détection de la présence d'anticorps dans l'échantillon de sérum lié au support.